Europäisches Patentamt

European Patent Office

Office européen des brevets

(19)

(11) Numéro de publication : **0 115 734 B1**

(12) **FASCICULE DE BREVET EUROPÉEN**

(45) Date de publication du fascicule du brevet :
02.03.88

(51) Int. Cl.⁴ : **H 01 J 47/06, A 61 B 6/02**

(21) Numéro de dépôt : **83402559.5**

(22) Date de dépôt : **29.12.83**

(54) **Procédé d'examen de l'image radiographique d'un objet irradié à l'aide d'une source de rayonnements ionisants et chambre d'ionisation pour la mise en oeuvre du procédé.**

(30) Priorité : **04.01.83 FR 8300034**

(43) Date de publication de la demande :
**15.08.84 Bulletin 84/33**

(45) Mention de la délivrance du brevet :
**02.03.88 Bulletin 88/09**

(84) Etats contractants désignés :
**DE GB NL**

(56) Documents cités :
**EP-A- 0 046 244**
**EP-A- 0 059 700**
**GB-A- 2 057 184**

(73) Titulaire : **COMMISSARIAT A L'ENERGIE ATOMIQUE**
**31/33, rue de la Fédération**
**F-75015 Paris (FR)**

(72) Inventeur : **Allemand, Robert**
**17, Chemin de Bouffière**
**F-38330 Saint Ismier (FR)**
Inventeur : **Gagelin, Jean-Jacques**
**"l'Allegrerie"**
**F-38470 Vinay (FR)**
Inventeur : **Pleyber, Gaetan**
**49, rue République**
**F-38420 Domene (FR)**

(74) Mandataire : **Mongrédien, André et al**
**c/o BREVATOME 25, rue de Ponthieu**
**F-75008 Paris (FR)**

## Description

La présente invention a trait à l'examen de l'image radiographique d'un objet obtenue à l'aide de rayonnements ionisants en provenance d'une source, ledit objet présentant dans l'étendue de son volume des zones de transparence variable audit rayonnement ionisant.

Dans les techniques connues de ce genre, on utilise généralement pour détecter l'intensité des rayonnements ionisants ayant traversé chaque élément de volume de l'objet à radiographier, une chambre d'ionisation dans laquelle lesdits rayonnements provoquent la naissance de charges électriques de signes contraires dont la localisation et l'intensité sont caractéristiques des propriétés absorbantes de l'objet étudié et constituent ce que l'on peut appeler une image électrique latente de l'objet ainsi examiné.

Cette image latente est donc formée d'un certain nombre de charges électriques réparties dans le volume de détection d'une chambre d'ionisation généralement remplie d'un gaz ionisable et possède la propriété d'être rapidement effacée puisque les charges ainsi formées (électrons et ions) se déplacent immédiatement, dès leur apparition, vers les électrodes collectrices de la chambre que constituent l'anode et la cathode de celle-ci. Le problème de l'examen de l'image latente ainsi obtenue est donc délicat à résoudre, d'autant plus que dans les procédés connus actuellement on travaille généralement à l'aide de flashes de rayonnements ionisants (rayons X, gamma ou neutrons, moyennant un convertisseur approprié) de durée très brève, ce qui diminue d'autant l'intensité des charges créées dans le volume de la chambre.

Les charges ainsi créées par ionisation produisent par influence sur chaque électrode des charges de signes opposés et de valeur égale qui, transformées en courant électrique détectable, donnent immédiatement une valeur globale de la somme des charges instantanées constituant l'image latente. Ceci malheureusement ne permet pas d'atteindre la cartographie souhaitable de l'image latente selon les deux coordonnées OX et OY respectivement parallèle et perpendiculaire au champ électrique de la chambre.

Pour pallier cette difficulté, on a imaginé de réaliser des chambres d'ionisation sous la forme de mosaïques de plusieurs chambres élémentaires juxtaposées, ce qui permet ainsi de repérer selon les deux coordonnées précédentes la répartition des charges spatiales induites par ionisation à partir du faisceau ionisant, mais l'existence des cloisons entre les différentes chambres adjacentes constitue une limitation importante de la précision de la détection et, de plus, la résolution spatiale d'un tel appareil est évidemment limitée par les dimensions du volume élémentaire de chaque chambre de détection.

On a également imaginé d'utiliser des procédés de collection des charges électriques formées sur des plaques isolantes sur lesquelles on obtient ainsi l'image latente à deux dimensions ; on peut ensuite lire cette image par des dispositifs électriques adéquats ou la révéler à la manière d'une plaque photographique par une lecture optique du type de celles que l'on utilise en xérographie.

D'une façon générale, l'état de la technique précédemment rappelé conduit à des inconvénients majeurs, qui rendent pratiquement impossible l'examen de l'image radiographique d'un objet irradié à l'aide d'une source de rayonnements ionisants en utilisant comme détecteur une chambre d'ionisation.

En effet, les dispositifs de chambre d'ionisation dites en mosaïque ont une résolution spatiale limitée par la taille des cellules élémentaires juxtaposées et sont d'une réalisation complexe donc coûteuse ; les dispositifs à collection de charges sur plaques isolantes nécessitent obligatoirement l'emploi de dispositifs spéciaux de mesure et de localisation de ces charges sur la surface et dans l'état actuel de la technique ces procédés sont extrêmement lents.

La présente invention a précisément pour objet un procédé d'examen de l'image radiographique d'un objet irradié à l'aide d'une source de rayonnement ionisant qui utilise une chambre d'ionisation et permet à la fois de former une image latente de l'objet examiné de très bonne qualité et d'en faire l'examen électrique précis par des moyens dont la mise en œuvre est simple.

Ce procédé d'examen de l'image radiographique plane d'un objet irradié à l'aide d'une source de rayonnements ionisants du genre de ceux dans lesquels on détecte dans une chambre d'ionisation l'image latente de l'objet formée par les différentes charges électriques naissant dans le volume de la chambre sous l'influence de l'impact du flux de rayonnement ayant traversé l'objet, la direction de ce flux étant distincte de celle du champ électrique de la chambre, les coordonnées x et y de chaque point de l'image étant déduites, pour la coordonnée x correspondant à la direction du déplacement de l'objet de l'époque d'arrivée de la charge correspondante sur l'électrode collectrice, et pour la coordonnée y perpendiculaire, de l'emplacement de l'impact de la même charge sur l'électrode collectrice, se caractérise en ce que l'irradiation étant continue on déplace l'objet à examiner par rapport à la chambre d'ionisation dans le sens et à la vitesse de migration vers l'électrode collectrice des ions formés dans le champ électrique de la chambre de façon à ce que les différentes images latentes naissant à chaque instant se superposent en une seule et même image qui s'enrichit ainsi au fur et à mesure d'informations nouvelles en se déplaçant à la vitesse des ions vers l'électrode collectrice.

On voit ainsi que grâce au déplacement de l'objet irradié à la vitesse des ions par rapport à la chambre, et à l'irradiation en continu de l'objet à examiner, on parvient à concentrer les différentes informations apparaissant au cours du temps sur la transparence de cet objet sur une seule et même image latente qui se déplace, par définition, en même temps que l'objet. Le fait que l'image latente est unique et reste la même au fur et à mesure du déplacement de l'objet, permet d'obtenir cette image en rayons X ou gamma en temps réel et de pallier tous les inconvénients qui résultaient dans l'art antérieur du fait que la réponse d'une chambre d'ionisation n'est pas uniforme en fonction du lieu d'apparition de chacune des charges, car il est bien connu que le pouvoir séparateur d'une chambre d'ionisation se dégrade au fur et à mesure qu'augmente l'éloignement du point d'apparition d'une charge par rapport à l'électrode collectrice. Selon le procédé objet de l'invention au contraire, l'image latente étant unique et se déplaçant à la vitesse des ions formés, on rétablit ainsi en quelque sorte l'uniformité de la réponse de la chambre puisque le temps de pose du système correspond au temps de déplacement de l'image latente ou de l'objet et qu'il est le même pour tous les motifs de l'image.

Par ailleurs, il est relativement facile de déplacer l'objet à examiner à la vitesse de migration des ions car dans une chambre d'ionisation courante, remplie par exemple de gaz xénon, cette vitesse de migration des ions est couramment de l'ordre de 100 cm/s, ce qui correspond approximativement à la vitesse d'un homme au pas. Cette vitesse V des ions est d'ailleurs fonction de trois paramètres que sont la mobilité $\mu_e$, le champ électrique E régnant entre l'anode et la cathode et la pression P du gaz de remplissage selon la formule :

$$V = \mu_e \, (E/P)$$

On peut ainsi facilement, en jouant sur le champ électrique E ou sur la pression P, régler à une valeur déterminée la vitesse des ions, et on peut même, conformément à l'invention, réaliser en cas de besoin un asservissement de la vitesse de déplacement de l'objet à la vitesse des ions précédente.

Enfin, il doit être bien entendu, ce qui est évident pour tout homme de l'Art, que le déplacement de l'objet par rapport à la chambre d'ionisation est à considérer comme un déplacement relatif, ce qui signifie que l'on reste dans le cadre du procédé objet de l'invention lorsque l'on déplace, à la vitesse de migration des ions, la chambre par rapport à l'objet restant fixe.

La présente invention a également pour objet une chambre d'ionisation pour la mise en œuvre du procédé précédent comprenant de façon connue une anode et une cathode planes se faisant face dans une atmosphère de gaz ionisable, caractérisée en ce qu'elle comprend, portée à un potentiel intermédiaire entre ceux de l'anode et de la cathode, une grille écran située dans la proximité immédiate de l'électrode collectrice des ions et en ce que cette électrode collectrice est divisée selon la direction de la coordonnée y perpendiculaire au champ, en plusieurs secteurs adjacents portés au même potentiel mais mécaniquement indépendants les uns des autres.

Grâce à la structure de la chambre d'ionisation précédente, on réalise simultanément deux objectifs différents qui sont expliqués ci-après.

D'abord, en plaçant dans la chambre d'ionisation une grille écran située le plus près possible de l'électrode collectrice des ions, on divise l'espace de la chambre en deux volumes inégaux, à savoir un volume de détection des informations portées par le flux de rayonnement ionisant qui correspond à l'espace compris entre la grille écran et l'électrode non collectrice et d'autre part un volume très petit de lecture de ces informations qui correspond à la partie de la chambre située entre la grille écran et l'électrode collectrice des ions. Le rôle de la grille écran est de constituer un véritable écran électrostatique qui permet précisément de séparer électrostatiquement les deux espaces précédents, c'est-à-dire que l'espace de lecture n'est influencé que par les charges qui s'y trouvent à la suite de leur migration, à l'exclusion de la grande majorité d'entre elles qui sont formées dans l'espace de détection beaucoup plus important situé entre la grille écran et l'électrode non collectrice. Selon l'invention, la grille écran est située à proximité immédiate de l'électrode collectrice des ions, c'est-à-dire aussi proche que possible mécaniquement parlant de celle-ci. Une distance optimale réalisable est par exemple de l'ordre de quelques dixièmes de mm. Cette même grille écran est portée à un potentiel intermédiaire entre celui de l'anode et celui de la cathode de la chambre ; ce potentiel est choisi expérimentalement ou par le calcul en fonction de la forme de la grille, de façon à rendre compatible le rôle d'écran électrostatique joué par celle-ci et sa relative bonne transparence aux ions qu'elle ne doit évidemment pas collecter en trop grand nombre en les empêchant de migrer de l'espace de détection vers l'espace de lecture.

La deuxième caractéristique adoptée par la chambre d'ionisation objet de la présente invention, est d'avoir une électrode collectrice des ions, le plus souvent d'ailleurs la cathode, divisée selon la direction Oy, perpendiculaire à la migration de ceux-ci, en plusieurs secteurs adjacents portés en parallèle au même potentiel électrique mais matériellement indépendants les uns des autres, ce qui assure une détection de la localisation de chaque charge électrique selon la coordonnée d'espace OY parallèle au plan de l'électrode collectrice. Selon la mise en œuvre du procédé objet de l'invention, l'image latente de l'objet irradié se déplace à la même vitesse que l'objet et par conséquent ceci permet, à partir de l'époque d'arrivée d'une charge donnée dans la bande de lecture étroite située entre la grille écran et l'électrode collectrice, de déterminer la coordonnée d'espace de cette même charge selon la direction OX du champ électrique de la chambre, c'est-à-dire selon la direction de migration des ions.

Enfin, selon l'invention, la chambre d'ionisation peut revêtir toute forme générale souhaitable en fonction de la source de rayonnement utilisée et de la répartition spatiale de celui-ci. C'est ainsi qu'elle peut être par exemple de forme générale parallélépipédique lorsque la source est suffisamment éloignée, le flux de rayonnement parvenant alors avec un relatif parallélisme sur l'objet à étudier ou qu'elle peut avoir une symétrie sphérique ou cylindrique centrée sur la source ponctuelle lorsque celle-ci est plus rapprochée, ce qui conduit alors à adopter pour le volume d'ionisation une forme en berceau.

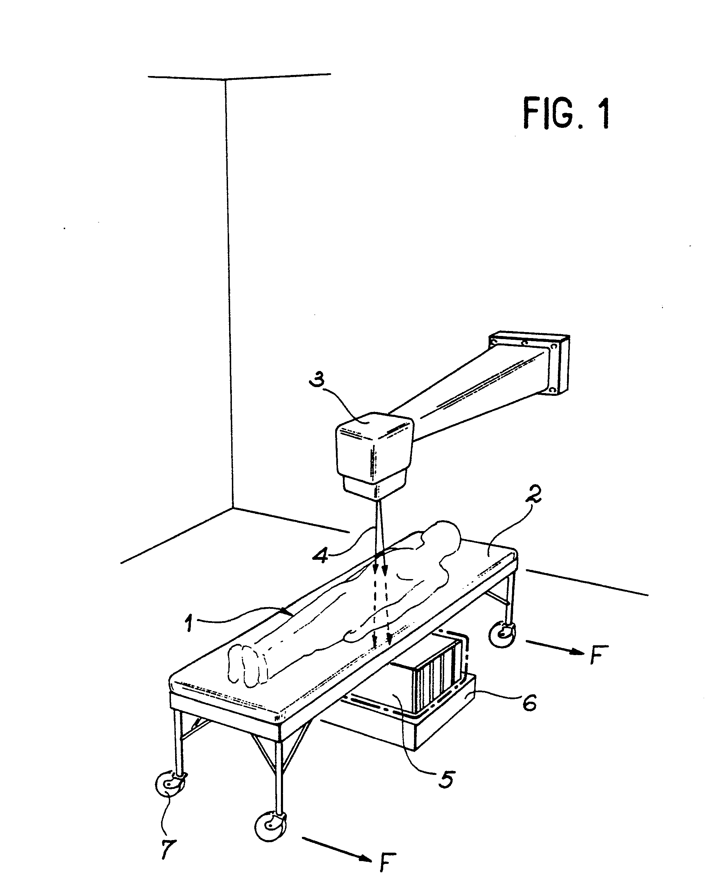

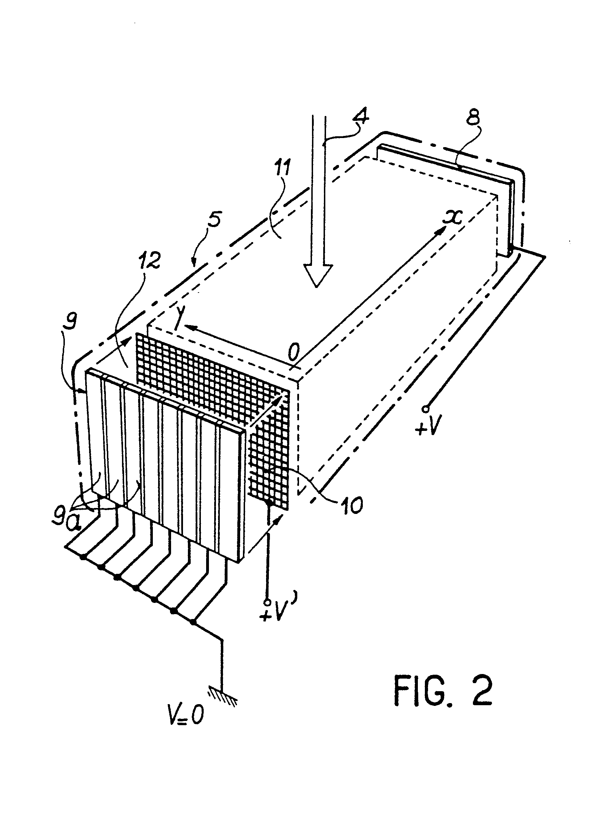

De toute façon, l'invention sera mieux comprise en se référant à la description qui suit de plusieurs exemples de mise en œuvre, exemples qui seront donnés à titre illustratif et non limitatif en se référant aux figures 1 et 2 ci-jointes sur lesquelles :

- la figure 1 représente une application du procédé d'examen de l'image radiographique selon l'invention appliquée à la radiographie du corps humain ;

- la figure 2 représente la structure d'une chambre d'ionisation objet de l'invention dans une configuration géométrique parallélépipédique.

Sur la figure 1, on a décrit un exemple d'application du procédé d'examen de l'image radiographique d'un objet irradié à l'aide de rayonnements ionisants dans lequel l'objet est un patient 1 allongé sur un lit mobile 2. Il doit être bien entendu que cet exemple est donné à titre illustratif de l'un des cas d'application intéressants du procédé objet de l'invention, mais que celle-ci s'applique de façon très générale à la radiographie d'un objet quelconque, dès lors qu'il présente vis-à-vis d'un flux de rayonnement ionisant X, gamma ou neutrons, une transparence variable caractéristique de sa structure interne que l'on veut examiner.

Sur la figure 1, une source 3 de rayonnement ionisant 4 irradie le patient 1 et une image radiographique est lue sous la table 2 par une chambre d'ionisation 5 fixée sur le sol à l'aide d'un socle 6. Conformément à l'invention, le lit 2 supportant le patient est mobile grâce à un système de roulettes 7 dans la direction des flèches F correspondant à celle du champ électrique de la chambre d'ionisation 5 à la vitesse de migration des ions formés dans l'espace de détection de la chambre 5 sous l'effet du rayonnement 4 ayant traversé le corps du patient.

Les moyens pratiques de mise en mouvement de la table 2 sur les roulettes 7 ne sont pas représentés de façon concrète sur la figure 1, mais il est bien évident que de tels moyens doivent être considérés comme étant à la portée de l'homme de l'art et que, comme expliqué précédemment en particulier, un système d'asservissement d'un type en soi connu peut également réaliser le synchronisme nécessaire entre la vitesse de déplacement de la table 2 et la vitesse de migration des ions dans la chambre d'ionisation 5.

Sur la figure 2, on a représenté schématiquement une chambre d'ionisation pour la mise en œuvre du procédé de l'invention dans une structure de forme parallélépipédique. Cette chambre d'ionisation comporte une anode 8 portée au potentiel + V et une cathode 9 au potentiel du sol constituée selon l'invention par la juxtaposition d'une série d'électrodes collectrices 9a portées toutes au même potentiel mais indépendantes les unes des autres matériellement parlant. Cette structure particulière de la cathode 9 conduit à réaliser dans la chambre d'ionisation 5 une juxtaposition immatérielle, c'est-à-dire sans cloisons, d'un certain nombre de chambres élémentaires en nombre égal à celui des électrodes collectrices 9a.

Selon l'invention, la chambre 5 comporte également une grille écran 10 portée à un potentiel V' intermédiaire entre ceux de l'anode 8 et de la cathode 9 et située à quelques dixièmes de millimètres de cette dernière. Dans un mode de réalisation préférentiel de la chambre 5, l'espace interne de celle-ci est rempli de gaz xénon et les potentiels V et V' sont respectivement de + 2 500 V et de + 400 V, l'espace entre la grille écran 10 et les électrodes collectrices 9a étant de 4/10 mm. La grille écran 10 est réalisée par un treillis de fils métalliques disposés selon deux directions perpendiculaires.

Selon l'invention, la grille écran 10 divise l'espace interne de la chambre 5 en deux zones différentes, à savoir : une première zone ou espace de détection 11 comprise entre la grille écran 10 et l'anode 8 et une deuxième zone ou espace de lecture 12 comprise entre la grille écran 10 et les électrodes collectrices 9a. On voit bien que du point de vue électrostatique les deux espaces 11 et 12 sont totalement séparés, ce qui leur permet de jouer chacun un rôle différent. En effet, l'espace 11 soumis au flux de rayonnement ionisant 4 schématisé par une flèche sur la figure 2 correspond à l'espace de détection, c'est-à-dire dans lequel de façon classique connue des rayonnements ionisants 4 créent, par ionisation du gaz xénon contenu dans la chambre 5, des paires d'ions positifs et d'électrons de charges égales et opposées qui migrent aussitôt dans la direction OX du champ électrique vers la cathode 9 et l'anode 8 ; l'espace de lecture 12 qui, pour des raisons de commodité de lecture a été très exagérément grossi sur la figure 2, puisqu'il ne fait que quelques dixièmes de millimètres en réalité, est celui dans lequel les charges ayant migré en provenance de l'espace 11 sont détectées par influence sur les diverses électrodes 9a au fur et à mesure qu'elles traversent la grille écran 10. L'apparition d'un signal électrique sur l'une de ces électrodes sectorielles 9a permet de déterminer la coordonnée OY perpendiculaire au champ électrique de la chambre 5 correspondant à la charge ainsi détectée.

Les chambres d'ionisation selon l'invention, précédemment décrites, possèdent des avantages particuliers non négligeables et notamment une grande simplicité de construction et un coût modeste pour une précision et un pouvoir de résolution très élevé.

Dans l'utilisation principale qui est celle de la prise d'image de la projection d'un objet irradié par une source ponctuelle de rayonnement X ou gamma, l'invention offre un système possédant une grande dynamique de mesure et une très grande uniformité de réponse. On notera également que les chambres d'ionisation conformes à l'invention ont un faible taux de distorsion géométrique résultant du quasi parallélisme des trajectoires des ions dans le milieu détecteur de la chambre.

Parmi les applications intéressantes de la présente invention, on peut citer l'électroradiographie X ou gamma numérique en continu, et notamment le contrôle d'objets disposés sur un tapis roulant (contrôle des bagages dans les aéroports par exemple), ou encore la tomographie longitudinale.

**Revendication**

Procédé d'examen de l'image radiographique plane d'un objet (1) irradié à l'aide d'une source (3) de rayonnements ionisants (4) du genre de ceux dans lesquels on détecte dans une chambre d'ionisation (5) l'image latente de l'objet (1) formée par les différentes charges électriques naissant dans le volume de la chambre sous l'influence de l'impact du flux de rayonnement ayant traversé l'objet (1), la direction de ce flux étant distincte de celle du champ électrique de la chambre, les coordonnées x et y de chaque point de l'image étant déduites, pour la coordonnée x correspondant à la direction du déplacement de l'objet (1), de l'époque d'arrivée de la charge correspondante sur l'électrode collectrice (9), et pour la coordonnée y perpendiculaire, de l'emplacement de l'impact de la même charge sur l'électrode collectrice (9), caractérisé en ce que l'irradiation étant continue, on déplace l'objet à examiner (1) par rapport à la chambre d'ionisation (5) dans le sens et à la vitesse de migration vers l'électrode collectrice (9) des ions formés dans le champ électrique de la chambre de façon à ce que les différentes images latentes naissant à chaque instant se superposent en une seule et même image qui s'enrichit ainsi au fur et à mesure d'informations nouvelles en se déplaçant à la vitesse des ions vers l'électrode collectrice.

**Claim**

Process for the examination of a flat radiograph of an object (1), irradiated with the aid of a source (3) of ionizing radiation (4) of the type in which detection takes place in an ionization chamber (5) of the latent image of the object (1) formed by the different electrical charges produced in the volume of the chamber under the influence of the impact of the radiation flux which has traversed the object (1), the direction of said flux being different to that of the electric field of the chamber, the x and y coordinates of each point of the image being deduced, for the x coordinate corresponding to the displacement direction of the object (1), from the arrival time of the corresponding charge on the collecting electrode (9), characterized in that, as the irradiation is continuous, the object (1) to be examined is moved relative to the ionization chamber (5) in the direction and at the migration speed of the ions formed in the electrical field thereof, in such a way that the different latent images formed at each instant are superimposed into a single image, which is thus enriched with new informations by moving at the speed of the ions towards the collecting electrode.

**Patentanspruch**

Untersuchungsverfahren des radiographischen, ebenen Bildes eines mit Hilfe einer Quelle (3) ionisierender Strahlung (4) bestrahlten Gegenstands von der Art, bei dem man in einer Ionisationskammer (5) das latente Bild des Gegenstands (1) erfaßt, welches von den verschiedenen elektrischen Ladungen in dem Kammervolumen unter dem Einfluß des Stoßes des durch den Gegenstand (1) hindurchgegangenen Strahlungsflusses gebildet wird, wobei die Richtung dieses Flusses von derjenigen des elektrischen Feldes der Kammer unterschiedlich ist, die Koordinaten x und y eines jeden Bildpunkts im Hinblick auf die der Bewegungsrichtung des Gegenstands (1) entsprechenden Koordinate x von dem Ankunftszeitpunkt der entsprechenden Ladung an der Sammelelektrode (9) und im Hinblick auf die senkrechte Koordinate y von dem Auftreffort der gleichen Ladung auf der Sammelelektrode (9) abgeleitet werden, dadurch gekennzeichnet, daß man bei kontinuierlicher Bestrahlung den zu untersuchenden Gegenstand (1) im Bezug auf die Ionisationskammer (5) in der Richtung und mit der Wanderungsgeschwindigkeit der in dem elektrischen Feld der Kammer gebildeten Ionen zu der Sammelelektrode (9) derart bewegt, daß sich die unterschiedlichen, latenten, zu jedem Zeitpunkt entstehenden Bilder zu ein und demselben Bild überlagern, welches sich somit nach und nach bei seiner Bewegung mit der Ionengeschwindigkeit zu der Sammelelektrode mit neuen Informationen anreichert.

FIG. 1

FIG. 2

0 115 734

2